# NEUE EUROPÄISCHE PATENTSCHRIFT

(11) **EP 1 014 916 B2**
(45) Veröffentlichungstag und Bekanntmachung des Hinweises auf die Entscheidung über den Einspruch: **15.06.2011**
(45) Hinweis auf die Patenterteilung: 19.12.2001
(21) Anmeldenummer: 98943893.2
(22) Anmeldetag: 18.08.1998
(51) Int. Cl.: A61K 8/00

(54) **SCHAUM-HAUTSCHUTZCREME**
FOAMING SKIN CREAM, USES THEREOF AND A METHOD FOR PRODUCING THE SAME
CREME MOUSSANTE DE SOINS CORPORELS, UTILISATIONS ET PROCEDE DE PRODUCTION DE CETTE CREME

(30) Priorität: 18.08.1997 DE 19735591
(43) Veröffentlichungstag der Anmeldung: 05.07.2000
(73) Patentinhaber: Neubourg Skin Care GmbH & Co. KG, 48268 Greven (DE)
(72) Erfinder: NEUBOURG, Fritz, D-48282 Emsdetten (DE)
(74) Vertreter: Maiwald Patentanwalts GmbH
(86) Internationale Anmeldenummer: PCT/EP1998/005232
(87) Internationale Veröffentlichungsnummer: WO 1999/008649

(56) Entgegenhaltungen:
- EP-A- 0 194 097
- EP-A- 0 257 336
- EP-A- 0 598 412
- DE-A- 3 330 628
- DE-A- 4 431 365
- FR-A- 2 217 405
- US-A- 2 968 628
- US-A- 4 661 340
- ZIOLKOWSKY: "Moderne Aerosolschaüme in der Kosmetik" SEIFEN-ÖLE-FETTE-WACHSE, Bd. 112, Nr. 13, August 1986, Seiten 427-429, XP002093534 augsburg, DE

## Beschreibung

Die Erfindung betrifft eine Schaum-Hautcreme.

Das galenische Prinzip eines emulsoiden 2-Phasen-Systems durch lipophile bzw. hydrophobe Anteile einerseits sowie hydrophile Komponenten andererseits, ist zur Herstellung von Schaumpräparaten für die Hautpflege bereits bekannt. Durch Anwendung dieser Schaumpräparate auf der behandelten Haut entsteht ein zweidimensionales zweiphasiges Netzwerk, die hydrophilen Komponenten binden sich an das Keratin der Hornschicht und gestatten die Abdunstung des Schweisses, während die lipophilen Anteile an der Hautoberfläche das Eindringen von Feuchtigkeit (einschließlich des durchgetretenen Schweisses) inhibieren. Da der Schaum im Unterschied zu anderen, eine Barriere aufbauenden Cremes in Minutenfrist einzieht, keine Fettspuren auf den Arbeitsmaterialien hinterläßt und Wärmestau oder sogar Mazerationseffekte durch Schweißeinwirkung unterbindet, erfreute sich der Schaum rasch zunehmender Beliebtheit als Schutzmittel vor geruchsbedingter Feuchtigkeitseinwirkung. Weitere Einzelheiten zu den an sich bekannten Schaum-Hautcremes sind insbesondere aus "Haut", Heft 4, 1992 von R. Rudolph, L. Bade, B. Brüggemann, entnehmbar.

In "hautnah derm" 10 (1994), 344 - 351 berichtet B. Kunze über fetthaltige Hautschutzschäume, die bei trockener empfindlicher Haut und chronisch-rhagadiformen Ekzemen indiziert sind. Es werden dort auch Inhaltsstoffe offenbart. Die dort beschriebenen Hautschutzschäume schützen gegen viele berufliche Noxen, wie z. B. saure Dauerwellflüssigkeiten im Friseurhandwerk, Laugen, Öle, Desinfektionsmittel, Reinigungs- und Spülmittel, aber auch gegen Wasser, Feuchtigkeit, Schweiß, Kot, Urin und mineralische Stäube.

Die EP 0 598 412 betrifft ebenfalls Hautschutzschäume, die als wirksames Prinzip PTFE beschreiben.

Die DE-C-33 30 628 betrifft Hautschutz- und Pflegelotionen, die Siliconöle, partiell neutralisierte Stearinsäure, Fettalkohole und deren ethoxylierte Derivate, ethoxylierte Wollfettalkohle, Cetylstearylalkohol, Vaseline, Verdicker und Wasser enthält. Die dort offenbarten Lotionen sollen schnell einziehen, ohne auf der Haut längere Zeit einen lästigen Fettfilm zu hinterlassen.

Angaben zu Emulgatoren, die in Hautpflegemitteln eingesetzt werden können, finden sich in der DE-A-195 42 572. Dort werden Emulgatoren vorgeschlagen, die 43 bis 90 Gew.-% Alkyl- und/oder Alkenyloligoglycoside und 10 bis 57 Gew.-% Fettalkohole enthalten. Diese Emulgatoren eignen sich insbesondere zur Herstellung von lagerstabilen hochviskosen, sensorisch leichten Öl-in-Wasser-Emulsionen. Das deutsche Gebrauchsmuster DE-U-9308050 betrifft einen Hautschutzschaum als Mittel gegen hautaggressive Mittel. Dieser Schaum besteht aus einer wäßrigen Emulsion, die Fettsäureester mit guter Hautverträglichkeit und zur Herstellung eines wasserdampfdurchlässigen Films, mehrwertigen Alkohol zur stabilen Dispersion der Wirkstoffe und Regulierung der Feuchtigkeit des Films, Emulgatoren zur Verbesserung der Schaumstabilität, Tensiden zur Verminderung der Oberflächenspannung und gegebenenfalls Neutralisationsmittel für Harze und Emulgatoren enthält. Als Treibmittel können gasförmige Kohlenwasserstoffe verwendet werden, wie beispielsweise Propan, Butan oder Isobutan sowie deren Mischungen. Die dort beschriebenen Schäume enthalten jedoch keine freien Fettsäuren.

Aerosole sind komplizierte, physikalisch-chemische Gebilde, die sich nicht nach Beliebigkeit bilden. Es kommt dabei insbesondere auf eine besondere Abstimmung der den Schaum bildenden Komponenten an. Dabei können bereits geringfügige Verschiebungen der Zusammensetzung zum Kollabieren des Schaumes führen, wodurch eine Abmischung an sich wirksamer Substanzen nicht ohne weiteres als Schaum formuliert werden kann.

Hautcremes werden oft, insbesondere bei Anwendungen im Beruf, als unangenehm empfunden, da dort zu lange die "fettenden" Komponenten auf der Haut verbleiben und zu lästigen Fingerspuren oder ganz allgemein auch zu einem unangenehmen Gefühl führen. Eine gewisse Abhilfe haben hier bereits die an sich bekannten Hautschäume geschaffen, da diese eine Überladung der Haut mit den fettenden Stoffen verhindern oder vermindern helfen. Nichtsdestoweniger ist es wünschenswert, insbesondere bei einer dauerhaften Anwendung von Hautschutzmitteln, eine noch stärkere Akzeptanz beim Anwender zu finden.

Der vorliegenden Erfindung liegt das technische Problem zugrunde, das Anwendungsspektrum der Hautschutzschäume zu erweitern, indem eine Abmischung bereitgestellt wird, welche vom Anwender in starkem Maße akzeptiert wird, wobei eine Vielzahl von Zumischungen von anderen Stoffen erlaubt, ohne das zur Anwendung kommende Aerosol zu zerstören oder die Eigenschaften des Aerosols zu verschlechtern.

Überraschenderweise wird das der Erfindung zugrundeliegende technische Problem gelöst durch eine Grundmischung einer Schaum-Hautcreme mit den Merkmalen des Anspruchs 1. Bevorzugte Ausführungsformen der erfindungsgemäßen Schaum-Hautcreme betreffen die Unteransprüche 2 bis 4.

Die erfindungsgemäße Schaum-Hautcreme ist erhältlich durch
- Herstellung einer Phase I durch Aufschmelzen bei 75 °C einer Mischung enthaltend Fettsäuren, insbesondere C₁₂ - C₂₂ Fettsäuren, ggf. ungesättigte und/oder mehrfach ungesättigte Fettsäuren, Emulgatoren, Coemulgatoren, wie Triceteareth-4-phosphat,
gefolgt von einer dosierten Zugabe unter Rühren zu einer
- auf 75 °C temperierten Phase II, die aus einer wäßrigen Mischung enthaltend Moisturiser, wie Propylenglykol und/oder mehrwertige Alkohole, insbesondere Glycerin, Emulgatoren, wie Alkyl-Sarcosinate, und Allantoin sowie Harnstoff erhalten wird,
- wobei eine homogene Vermischung der Phasen I und II einzustellen ist und die dosierte Zugabe bei einer Temperatur von 75 °C erfolgt,
- nach Zugabe die Temperatur für eine Zeit zwischen 5 und 20 Minuten bei 75 °C gehalten wird, wonach
- die Temperatur der so erhaltenen Mischung unter ständigem Rühren auf eine Temperatur zwischen 30 und 40 °C heruntergefahren wird,
- Einstellung des pH-Wertes auf 7,6 bis 8,2, mit einer hautverträglichen basischen organischen Verbindung und Abfüllung der erhaltenen Mischung in Darreichungsformen unter Zugabe eines Treibgases.

Das gemäß dem Verfahren erhältliche Produkt kann zum einen direkt eingesetzt werden als Schaumhautcreme im gewerblichen Bereich zum Schutz vor oder Verminderung der Resorption von kanzerogenen Stoffen, wie polycyclischen, aromatischen Kohlenwasserstoffen. Diese Mischung ist bereits in der Lage bei Personen, deren Haut kanzerogenen Stoffen, wie polycyclischen aromatischen Verbindungen ausgesetzt ist, wirksamen Schutz zu verleihen. Desweiteren ist die Mischung geeignet, Personen vor der Einwirkung von Säuren, Laugen (nicht über pH 11), Spül- und Waschmittel, Schweiß, Urin, Stuhl, Gummihandschuhen, Stäuben, Hausstaub, Maschinen-, Bohr- und Kühlöle, Fetten, Farben/Lacken, Gips und anderen Substanzen und Chemikalien, insbesondere aggressiven Substanzen und Chemikalien, Schutz zu verleihen.

Die erfindungsgemäße Schaumhautcreme enthält Fettsäuren, insbesondere C₁₂ - C₂₂-Fettsäuren, die gegebenenfalls ungesättigte und/oder mehrfach ungesättigte Fettsäuren zusätzlich enthalten kann. Als Fettsäuren, die gesättigt sind, kommen insbesondere die natürlich vorkommenden C₁₂ - C₂₀-Fettsäuren, insbesondere Stearinsäure und Palmitinsäure in Betracht. Auch Myristinsäure ist einsetzbar. Als ungesättigte Fettsäuren kommen insbesondere solche mit bis zu drei ungesättigten Bindungen in der Kohlenwasserstoffkette in Betracht. Ungesättigte Fettsäuren finden sich beispielsweise in Fraktionen des Kokosfetts. Als Coemulgatoren kommen an sich bekannte Coemulgatoren, insbesondere Triceteareth-4-phosphat, Natriumlaureth-4-phosphat oder Oleth-3 sowie andere liphophile Emulgatoren auf Basis niedrig ethoxylierter Fettalkohole in Betracht.

Die Phase I kann desweiteren noch Paraffinum Liquidum enthalten. Als in Phase I einzusetzende Emulgatoren kommen solche auf Fettalkoholbasis und Basis von Partialestern von Fettsäuren in Betracht. Besonders bevorzugte Fettalkohole sind Cetearylalkohol. Als Partialester einer Fettsäure wird zum Beispiel Glycerylstearat eingesetzt.

Die in Phase II einsetzbaren Moisturiser sind insbesondere Propylenglykol und/oder mehrwertige Alkohole, wie Glycerin. Als in Phase II insbesondere einzusetzende Emulgatoren, sind Alkylsarcosinate, wie Lauroyl-, Lauryl, Cetyl-Sarcosinate zu nennen.

Die gemäß Anspruch 1 erhältliche Mischung dient auch als Grundlage für weitere Schaum-Hautcremes, die in weiten Bereichen zum Schutz der Haut und Linderung dermatologischer Fehlfunktionen eingesetzt werden können.

Gewünschtenfalls können der erfindungsgemäßen Schaum-Hautcreme ein oder mehrere Konservierungsmittel zugegeben werden. Als Konservierungsmittel haben sich insbesondere Stoffe wie Methyldibromoglutaronitril und/oder Phenoxyethanol bewährt. Diese Stoffe können in Mengen von 0,01 bis 1 Gew.-% zugegeben werden.

Bevorzugt werden die Emulgatoren, die Fettsäuren, Coemulgatoren, Moisturiser und Allantoin, in folgenden Mengen eingesetzt:
4 bis 15 Gew.-% Öl-in-Wasser Emulgator,
1 bis 10 Gew.-% Fettsäure, insbesondere 4 bis 7 Gew.-%, vorzugsweise 4,5 bis 6 Gew.-&
0,4 bis 2,3 Gew.-% Coemulgator
1 bis 10 Gew.-% Moisturiser
0,05 bis 1 Gew.-% Allantoin sowie
Wasser zum Ausgleich auf 100 % Gew.%.

In einer bevorzugten Ausführungsform weist die erfindungsgemäße Schaum-Hautcreme zusätzlich eine siliconhaltige Substanz, wie Dimethicon auf. Diese Substanz wird der Phase I zugegeben. Vorzugsweise liegt sie in Mengen von 0,05 bis 1 Gew.-% vor.

Die erfindungsgemäße Schaum-Hautcreme kann desweiteren zusätzlich in Phase I einen oder mehrere rückfettende Stoffe, wie Decyloleat, Isohexadecan, Stearinsäureglykolester, Kokosfettsäureethanolamid, Maisöl, Erdnußöl, Mandelöl, Sesamöl, Olivenöl, Jojobaöl, Sojaöl, Wollwachsalkohole, Paraffin, mittelkettige Triglyceride, Ölsäureoleylester, weißes Vaselin, Macrogol-Glycerolhydroxistearat, hydriertes Rizinusöl, Ricinusöl communis, Avocadoöl, Weizenkeimöl, Palmitinsäureisopropylester, Cetylpalmitat, Myristinsäuremyristilester und/oder Octyldodecanol aufweisen.

Insbesondere weist die Schaum-Hautcreme Mengen von 0,5 bis 2 Gew.-% Decyloleat und/oder 0,5 bis 2 Gew.-% Octyldekanol oder einen weiteren der genannten rückfettenden Stoffe auf, wenn das Produkt bei normaler Haut eingesetzt werden soll. Dabei soll die Gesamtmenge an rückfettenden Stoffen so bemessen sein, daß der Hautschutzschaum nicht zerstört wird. Eine Menge von etwa 4 Gew.-% an rückfettenden Stoffen ist ausreichend bei normaler bis leicht trockener Haut. Soll trockene Haut mit der erfindungsgemäßen Schaum-Hautcreme behandelt werden, empfehlen sich Mengen von 3 bis 6 GeW -% einer Komponente der rückfettenden Stoffe, wie insbesondere Decyloleat und/oder Octyldecanol. Werden mehr als eine Komponente eingesetzt, sollte die Gesamtmenge rückfettender Bestandteile bei leicht trockener Haut höchstens bei etwa 12% liegen. Wird noch stärker trockene Haut behandelt, liegt die Menge an rückfettender Substanz bei Verwendung einer Komponente wie Decyloleat bei etwa 6 bis 9 Gew.-% und bei Verwendung von Octyldecanol bei 6 bis 9 Gew.-%. Die Menge an rückfettender Substanz kann dann bis zu 20 Gew.-% betragen.

Die letztgenannten Schaum-Hautcremes eignen sich ebenso wie die Produkte ohne siliconhaltige Stoffe zur Pflege und zum Schutz der Haut, insbesondere gegenüber aggressiven Stoffen, wie Spül- und Waschmittel, Schweiß, Urin, Stuhl, Gummihandschuhen, Stäuben, Hausstaub, Maschinen-, Bohr- und Kühlöle, Fetten, Farben/Lacken, Gips und anderen Substanzen und Chemikalien, insbesondere aggressiven Substanzen und Chemikalien.

Die Schaum-Hautcremes eignen sich auch zur Behandlung von dermatologischen Erkrankungen oder deren Linderung. Dabei kommen insbesondere dermatologische Erkrankungen, wie allergisches Kontaktekzem vom Typ I und IV, kumulativ-subtoxisches Ekzem, toxisch-irritatives Ekzem, mikrobiell-dysregulatives Ekzem, atopische Dermatitis, atopisches PalmoPlantar-Ekzem, Dyshidrosis, Hyperhidrosis, Kontakturtikaria, intertriginöse Ekzeme bei Hämorrhoiden, diverse nässende Pilzinfektionen, z. B. Interdigitalmykose, Perlèches, Psoriasis vulgaris, Ulcus cruris, cholinerge Urticaria, Windeldermatitis in Betracht.

Die Menge an Harnstoff kann je nach Schweregrad der zu behandelnden Erkrankung 1 bis 20 Gew.-% oder mehr betragen, insbesondere von 3 bis 15 Gew.-%, oder 12,5 - 15 Gew.-%. Die erfindungsgemäße Schaum-Hautcreme kann ebenso bei Psoriasis eingesetzt werden.

Ein besonders wichtiges Anwendungsfeld erschließt sich bei der Prophylaxe und Behandlung des diabetischen Fußes. Aufgrund von Begleiterscheinungen der Diabetes leiden Diabetiker unter trockener Haut, die im Verlaufe der Erkrankungen die Schädigung der Extremitäten begünstigt, bis hin zur nötigen Amputation bei langjährigen Diabetikern. Eine prophylaktische Anwendung von Hautschutzmitteln, die für eine Verbesserung der Hautparameter bei Diabetikern sorgen, läßt sich der Ausbildung des diabetischen Fußes vorbeugen.

Desweiteren können mehrfach ungesättigte Fettsäuren (PUFA) zugegeben werden. Insbesondere kommen hierbei Omega-6-Fettsäuren, wie sie beispielsweise aus Nachtkerzenöl und Borretschöl bekannt sind, in Betracht. Dabei können sowohl synthetische Omega-6-Fettsäuren wie auch solche aus den genannten Pflanzenextrakten eingesetzt werden.

Desweiteren kommen reizlindernde Substanzen, nämlich Kamillenextrakt als weitere Fraktion, die der erfindungsgemäßen Schaum-Hautcreme zugemischt werden können, in Betracht.

Auch hautwirksame Vitamine, wie die Vitamine A, E und F können der erfindungsgemäßen Schaum-Hautcreme in wirksamen Mengen beigemischt werden. Insbesondere zur Behandlung und Prävention der Windeldermatitis hat sich die Zumischung der folgenden Stoffe bewährt, 3 - 7 Gew.-% Kamillen-Extrakt, 1 - 5 Gew.-% Decyloleat, 1 - 5 Gew.-% Octyldodecanol.

### Referenzbeispiel (nicht erfindungsgemäß)

Eine Hautschutzcreme wird in einer heiz- und kühlbaren geschlossenen Apparatur mit einem selbstaustragenden Homogenisator und einem heizbaren Dosiertrichter (vorzugsweise eine Koruma-Mehrzweck-Apparatur) hergestellt

Die Herstellung der Phase I erfolgt in einem beheizbaren Dosiertrichter durch Aufschmelzen einer Mischung enthaltend 2 Gew.-% Glycerylstearat, 4 Gew.-% Cetearylalkohol, 5 Gew.-% Stearinsäure, 1 Gew.-% Paraffin, 1 Gew.-% Triceteareth-4-phosphat bei 75°C. Es erfolgt eine dosierte Zugabe dieser Phase unter Rühren zu einer in der heiz- und kühlbaren geschlossenen Apparatur mit einem selbstaustragenden Homogenisator vorgelegten Phase II. Diese Phase besteht aus einer wäßrigen Mischung enthaltend 2,5 Gew.-% Propylenglycol, 2,5 Gew.-% Glycerin, 2 Gew.-% Natriumlauroylsarcosinat sowie 0,3 Gew.-% Allantoin. Die Menge des Wasser 79,7 Gew.-%. Eine homogene Vermischung der Phasen I und II ist einzustellen.

Die dosierte Zugabe der Phase I erfolgt bei einer Temperatur von 75°C. Beide Phasen werden unter ständiger mittlerer Rührgeschwindigkeit zusammengeführt, wobei auf eine gleichmäßige Homogenisierungstätigkeit geachtet werden muß; Die Temperatur wird zwischen 5 und 20 min. bei 75°C gehalten. Die erhaltene Mischung wird unter ständigem Rühren auf eine Temperatur zwischen 30 und 40°C abgekühlt.

Nach Erreichen einer Temperatur von 40°C können dann die weiteren Stoffe zugesetzt werden. Dabei kann auch der pH-Wert. auf einen Wert zwischen 7,8 bis 8,0 eingestellt werden.

Hierzu wird 2-Amino-2-methyl-1-propanol eingesetzt. Es wird für eine hinreichend lange Zeit bis zur Stabilisierung des pH-Wert gerührt und danach die Abfüllung in geeignete Lagerbehälter bzw. in die entsprechenden Sprühbehälter abgefüllt. Dabei werden 91% Wirkstoff mit 9% Butan/Propan abgefüllt.

## Patentansprüche

1. Schaum-Hautcreme bestehend aus
Fettsauren,
Emulgatoren,
Coemulgatoren,
Wasser,
Moisturiser,
Harnstoff Allantoin,
Treibgas,
einer hautverträglichen basischen organischen Verbindung,
wehlweise Paraffinum liquidum;
wahlweise Konservierungsmittel(n),
wahlweise einer siliconhaltigen Substanz,
wahlweise einem oder mehreren rückfettenden Stoffen,
wahlweise reizlindernden Substanzen, nämlich Kamillenextrakt,
wahlweise hautwirksamen Vitaminen
erhältlich durch
- Herstellung einer Phase I durch Aufschmelzen bei 75 °C einer Mischung enthaltend Fettsäuren, insbesondere C₁₀ - C₂₂ Fettsäuren, ggf. ungesättigte und/oder mehrfach ungesättigte Fettsäuren, Emulgatoren, Coemulgatoren, wie
Triceteareth-4-phosphat, unter wahlweiser Zugabe von Paraffinum Liquidum, einer siliconhaltigen Substanz und/oder mehrerer rückfettender Stoffe,
gefolgt von einer dosierten Zugabe unter Rühren zu einer
- auf 75 °C temperierten Phase II, die aus einer wässrigen Mischung enthaltend Moisturiser, wie Propylenglykol und/oder mehrwertige Alkohole, insbesondere Glycerin Emulgatoren, wie Alkyl-Sarcosinate und Allantoin, sowie Harnstoff erhalten wird,
- wobei eine homogene Vermischung der Phasen I und II einzustellen ist und die dosierte Zugabe bei einer Temperatur von 75 °C erfolgt,
- nach Zugabe die Temperatur für eine Zeit zwischen 5 und 20 Minuten bei 75 °C gehalten wird, wonach
- die Temperatur der so erhaltenen Mischung unter ständigem Rühren auf eine Temperatur zwischen 30 und 40 C heruntergefahren wird, und die Mischung aus Phase I und II wahlweise unter Rühren bei 30° bis 40° C mit einem Konservierungsmittel versetzt wird,
- Einstellung des pH-Wertes auf 7,6 bis 8,2 mit einer hautverträglichen basischen organischen Verbindung, und Abfüllung der erhaltenen Mischung in Darreichungsformen unter Zugabe eines Treibgases.

2. Schaum-Hautcreme gemäß Ansprüch 1, enthaltend in Phase 1 Dimethicon als siliconhaltige Verbindung, vorzugsweise in Mengen von 0,05 bis 1 Gew.-%.

3. Schaum-Hautcreme gemäß einem der Ansprüche 1 und 2, enthaltend in Phase I einen oder mehrere rückfettende Stoffe, wie Decyloleat, Isohexadecan, Stearinsäureglykolester, Kokosfettsäureethanolamid, Maisöl, Erdnußöl, Mandelöl, Sesamöl, Olivenöl, Jojobaöl, Sojaöl, Wollwachsalkohole, Paraffin, mittelkettige Triglyceride, Ölsäureoleylester, weißes Vaselin, Macrogol-Glycerolhydroxistearat, hydriertes Rizinusöl, Ricinusöl communis, Avocadoöl, Weizenkeimöl, Palmitinsäureisopropylester, Cetylpalmitat, Myristinsäuremyristilester und/oder Octyldodecanol.

4. Schaum-Hautcreme gemäß einem der Ansprüche 1 bis 3, enthaltend mehrfachungesättigte Fettsäuren, wie ω-6-Fettsäuren, reizlindernde Substanzen, nämlich Kamillenextrakt, und/oder hautwirksame Vitamine.

## Claims

1. A foam skin cream consisting of
fatty acids,
emulsifiers,
coemulsifiers,
water,
moisturizers,
allantoin,
urea,
blowing gas,
a skin tolerable basic organic compound,
optionally paraffinum liquidum;
optionally preservative(s),
optionally a silicone-containing compound,
optionally one or more refatting substances,
optionally soothing substances, i.e. chamomile extract,
optionally skin-active vitamins
obtainable by
- preparing a phase I by melting at 75 °C a mixture containing fatty acids, especially C₁₀-C₂₂ fatty acids, optionally unsaturated and/or polyunsaturated fatty acids, emulsifiers, coemulsifiers, such as triceteareth-4-phosphate, and optionally adding paraffinum liquidum, a silicon-containing compound and/or multiple refatting substances,
followed by a dosed addition with stirring to
- a phase II temperature controlled to 75 °C which is obtained from an aqueous mixture containing moisturizers, such as propylene glycol and/or polyvalent alcohols, especially glycerol, emulsifiers, such as alkyl sarcosinates, and allantoin as well as urea,
- wherein a homogeneous mixing of phases I and II is to be adjusted, and said dosed addition is effected at a temperature of 75 °C,
- after the addition, keeping the temperature at 75 °C for a period of between 5 and 20 minutes, whereupon
- the temperature of the thus obtained mixture is controlled down to a temperature of between 30 and 40 °C with constant stirring, and optionally adding a preservative to the mixture of phase I and II with stirring at a temperature of 30 °C to 40 °C,
- adjusting the pH value to from 7.6 to 8.2 with a skin-tolerable basic organic compound, and filling the mixture obtained into dosage forms with the addition of a blowing gas.

2. The foam skin cream according to claim 1, containing in phase I dimethicon as silicone-containing compound, preferably in amounts of from 0.05 to 1% by weight.

3. The foam skin cream according to claim 1 or claim 2, containing in phase I one or more refatting substances, such as decyl oleate, iso-hexadecane, stearic acid glycol ester, coconut fatty acid ethanol amide, corn oil, peanut oil, almond oil, sesame oil, olive oil, jojoba oil, soybean oil, wool wax alcohols, paraffin, medium-chain triglycerides, oleic acid oleyl ester, white petrolatum, macrogol glycerol hydroxystearate, hydrogenated castor oil, common castor oil, avocado oil, wheat germ oil, palmitic acid isopropyl ester, cetyl palmitate, myristic acid myristyl ester, and/or octyldodecanol.

4. The foam skin cream according to any one of claims 1 to 3, containing polyunsaturated fatty acids, such as ω-6 fatty acids, soothing substances, i.e. chamomile extract, and/or skin-active vitamins.

## Revendications

1. Crème mousse pour la peau, constituée des composants suivants :
acides gras,
émulsionnants,
co-émulsionnants,
eau,
humectant,
urée,
allantoïne,
gaz propulseur,
composé organique basique bien toléré par la peau,
en option, de l'huile de paraffine ;
en option, un /des conservateur(s),
en option, une substance contenant de la silicone,
en option, une ou plusieurs substances relipidantes,
en option, des substances apaisant les irritations, à savoir de l'extrait de camomille,
en option, des vitamines agissant sur la peau,
pouvant être obtenue par
- la préparation d'une phase I par fusion à 75 °C d'un mélange contenant des acides gras, en particulier des acides gras en C₁₀-C₂₂, le cas échéant des acides gras insaturés et/ou polyinsaturés, des émulsionnants, des co-émulsionnants tels que du tricétéareth-4 phosphate, et, en option, par ajout de l'huile de paraffine, d'une substance contenant de la silicone et/ou de plusieurs substances relipidantes,
suivie, d'un ajout dosé, sous brassage, à
- une phase II portée à une température de 75 °C, qui est obtenue à partir d'un mélange aqueux contenant un humectant, tel que le propylène glycol et/ou des alcools polyvalents, en particulier du glycérol, des émulsionnants, tels que des alkylsarcosinates, et de l'allantoïne ainsi que de l'urée,
- en réalisant un mélange homogène des phases I et II et en effectuant un aj out dosé à une température de 75 °C,
- en maintenant, après ajout, la température pendant 5 à 20 minutes à 75 °C, après quoi
- on baisse la température du mélange obtenu de telle sorte à entre 30 et 40 °C, sous brassage continu, et que l'on additionne, en option, à une température de 30 à 40°C, un conservateur au mélange des phases I et II
- on ajuste le pH à 7,6 jusqu'à 8,2 à l'aide d'un composé organique basique bien toléré par la peau et que l'on réalise le remplissage du mélange obtenu pour conditionnements, sous addition d'un gaz propulseur.

2. Crème mousse pour la peau selon la revendication 1, contenant dans la phase I de la diméthicone comme composé contenant de la silicone, de préférence en quantités de 0,05 à 1 % en poids.

3. Crème mousse pour la peau selon l'une quelconque des revendications 1 et 2, contenant dans la phase I une ou plusieurs substances relipidantes, telles que de l'oléate de décyle, de l'isohexadécane, de l'ester de glycol et d'acide stéarique, de l'éthanolamide d'acides gras de l'huile de coco, de l'huile de maïs, de l'huile d'arachide, de l'huile d'amande, de l'huile de sésame, de l'huile d'olive, de l'huile de jojoba, de l'huile de soja, des alcools de lanoline, de la paraffine, des triglycérides à chaîne moyenne, de l'ester oléylique d'acide oléique, de la vaseline blanche, de l'hydroxystéarate de macrogolglycérol, de l'huile de ricin hydrogénée, de l'huile de ricin commune, de l'huile d'avocat, de l'huile de germe de blé, de l'ester isopropylique d'acide palmitique, du palmitate de cétyle, du myristate de myristyle et/ou de l'octyldodécanol.

4. Crème mousse pour la peau selon l'une quelconque des revendications 1 à 3, contenant des acides gras polyinsaturés, tels que des acides gras ω-6, des substances apaisant les irritations, telles que l'extrait de camomille, et/ou des vitamines agissant sur la peau.
